(19) Europäisches Patentamt
European Patent Office
Office européen des brevets

(11) EP 1 785 493 A1

(12) EUROPEAN PATENT APPLICATION

(43) Date of publication:
16.05.2007 Bulletin 2007/20

(51) Int Cl.:
C12Q 1/68 (2006.01)

(21) Application number: 06255175.9

(22) Date of filing: 06.10.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 18.10.2005 JP 2005302589

(71) Applicant: SONY CORPORATION
Tokyo 141 (JP)

(72) Inventors:
• Yamamoto, Takuro,
Sony Corporation
Shinagawa-ku,
Tokyo 141 (JP)

• Soma, Haruhiko,
Sony Corporation
Shinagawa-ku,
Tokyo 141 (JP)
• Takumi, Toru,
Sony Corporation
Shinagawa-ku,
Tokyo 141 (JP)
• Nakahata, Yasukazu,
Sony Corporation
Shinagawa-ku,
Tokyo 141 (JP)

(74) Representative: Robinson, Nigel Alexander Julian
et al
D Young & Co
120 Holborn
London EC1N 2DY (GB)

(54) Method for stress evaluation

(57) The molecular reaction mechanism that associates the clock gene with stress is elucidated based on the finding that stress is accompanied by an increase in the expression level of Per1 gene in peripheral tissues, stress response does not change the circadian rhythm in peripheral tissues, and Per1 gene increases in its expression level dependently on the responsive element of glucocorticoid existing in its promoter region. This finding is used for stress evaluation. That is, stress can be evaluated for its presence or absence or degree by detection and determination of an increase in the transcript (mRNA) of Per1 gene in peripheral tissues (an increase in the protein coded by Per 1 gene).

FIG.1A

EP 1 785 493 A1

## Description

The present invention relates to a method for stress evaluation which is intended to detect any increase in the expression level of Per1 gene in peripheral tissues, Per1 gene, PER1 which is a protein coded by Per1 gene, and use of the transcript of Per1 gene or the PER1 as a marker for stress detection.

All living organisms ranging from unicellular organisms to human beings ubiquitously have the circadian rhythm, which is an intravital phenomenon or a biological rhythm based on a 24-hour cycle. They also have a gene that controls the circadian rhythm.

In mammals, the circadian rhythm controlling gene expresses mostly in the suprachiasmatic nucleus (SCN) of the hypothalamus and it plays an important role in controlling the individual circadian rhythm. It expresses also in other tissues in the living organism, thereby controlling the circadian rhythm for cells and tissues in the same way as in the center.

It is known that the circadian rhythm controlling genes include Per genes (Per1, Per2, and Per3), Clock gene, Bmal genes (Bmal1, Bmal2, and Bmal3), Cry gene, Dec genes (Dec1 and Dec2), Dbp gene, E4Bp4 gene, and Rev-erb genes (Rev-erbα and Rev-erbβ).

The present invention is preceded by Japanese Patent Laid-open No. 2000-275248 which discloses a method for determining chronic stress by measurement of cortisol in saliva. Incidentally, the present invention employs Sokolove P.G. and Bushell W.N. (1978) J Theor Biol. 72, 131-160 as a reference for its examples of non-patent document mentioned later.

It is desirable to elucidate the molecular reaction mechanism that associates the clock gene with stress in view of the fact it has been left unclear unlike the circadian rhythm controlling mechanism relating to stress responsiveness which is believed to exist in mammals.

The present inventors completed this invention based on their finding that stress is accompanied by an increase in the expression level of Per1 gene in peripheral tissues, stress response does not change the circadian rhythm in peripheral tissues, and Per1 gene increases in its expression level dependently on the responsive element of glucocorticoid existing in its promoter region.

The foregoing finding suggests the existence of the following stress response mechanism. Any stress applied to an individual organism causes the adrenal cortex to secrete glucocorticoid for circulation into every part of the body. And the expression level of Per1 gene increases dependently on the responsive element of gluococorticoid existing in the promoter region. That is, stress increases the expression level of Per1 gene by a mechanism different from the one such as the expression of clock gene and the circadian rhythm.

The above-mentioned finding is useful for stress evaluation. That is, it would be possible to detect the presence or absence of stress or to evaluate the degree of stress by detecting the increase of or determining the amount of mRNA and PER1, the former being a transcript of Per1 gene in peripheral tissues, and the latter being a protein coded by Per1 gene.

Detection of a transcript of Per1 gene may be accomplished by synthesis of total RNA or extraction of total RNA from peripheral tissues and hybridization of polynucleotide (as a detection probe) having the base sequence (or part thereof) of Per1 with an extracted or synthesized nucleic acid. Also, detection of PER1 (protein) may be accomplished by the antigen-antibody reaction that involves the anti-PERI antibody that specifically connects with PER1.

Information about the base sequence of Per1 gene and the amino acid sequence due to Per1 gene is available from the gene database of NCBI (National Center for Biotechnology Information). As an example, sequence No. 1 shows the base sequence of homolog of human Per1 gene, and sequence No. 2 shows the base sequence of mouse Per1 gene. Per1 gene according to an embodiment of the present invention includes not only the sequence of homolog of Per1 gene in various animals but also those which partly have replacement, defect, insertion, and addition.

An embodiment of the present invention permits an objective and accurate evaluation of stress.

Embodiments of the invention will now be described, by way of example only, with reference to the accompanying drawings in which:

Fig. 1A is an actogram showing how a mouse changes its movement in one day under stress in Example 1;
Fig. 1B is a diagram which graphically represents the average values of the mouse's circadian rhythm observed in the actogram in Fig. 1A in Example 1;
Fig. 1C is a graph showing the change of activity ratio which was observed before and after stress application in Example 1;
Fig. 1D is a graph showing the number of days until resynchronization after stress application in Example 1;
Fig. 2A is a graph showing how the amount of expression of clock genes in the liver varies in a circadian cycle in Example 2;
Fig. 2B is a graph showing how the amount of expression of clock genes in the heart varies in a circadian cycle in Example 2;
Fig. 2C is a graph showing how the amount of expression of clock genes in the kidney varies in a circadian cycle in Example 2;
Fig. 3A is a graph showing the amount of expression of clock genes in the liver in Example 3;
Fig. 3B is a graph showing the amount of expression of clock genes in the heart in Example 3;
Fig. 3C is a graph showing the amount of expression of clock genes in the lung in Example 3;
Fig. 3D is a graph showing the amount of expression of clock genes in the kidney in Example 3;
Fig. 4A is a diagram showing the GRE resembling sequence and the sequence in its vicinity in Example 4;
Fig. 4B is a schematic diagram showing the position of each promoter on the sequence in Example 4;
Fig. 4C is a graph showing the results of luciferase assay in Example 4;
Fig. 4D is a graph showing the results of the luciferase assay when the GRE resembling sequence is changed;
Fig. 5A is a schematic diagram illustrating the sequence of Per1 gene in Example 5;
Fig. 5B is a photograph showing the electrophoresis for detection of the desired sequence by semi-quantitative RT-PCR which was performed on the immunoprecipitates obtained by ChIP method in Example 5;
Fig. 5C is a graph showing the result of the experiment to detect the desired sequence by quantitative real time RT-PCR that follows immunoprecipitation by ChIP method in Example 5;
Fig. 6A is a photograph showing the electrophoresis for detection of the desired sequence by semi-quantitative RT-PCR which was performed on the immunoprecipitates obtained by ChIP method in Example 6;
Fig. 6B is a graph showing the result of the experiment to detect the desired sequence by quantitative real time RT-PCR that follows immunoprecipitation by ChIP method in Example 6;
Fig. 7A is a graph showing the correlation between the dosage of corticosterone and the amount of expression of Per1 gene in Example 7;
Fig. 7B is a graph showing the amount of expression of each clock gene induced by administration of corticosterone (10 mg/kg) in Example 7; and
Fig. 8 is a schematic diagram showing the mechanism for stress response relating to an embodiment of the present invention.

Example 1

This example is intended to investigate how a mouse changes its movement in one day under stress by analyzing the data of mouse's movement recorded by an infrared movement recording apparatus (made by Biotex).
Mouse movement was recorded on an actogram by allowing a mouse of BALB/c strain to move freely for 24 hours in the dark in a cage equipped with an infrared movement recording apparatus after adaptation to the circadian rhythm of bright and dark environments at intervals of 12 hours (the former lasting from 8 to 20 o'clock) for 2 weeks. During its free run, the mouse was given a one-hour stress at prescribed times in consecutive two days. Incidentally, the actogram is a record showing the mouse's continuous movement. The resulting actogram was analyzed by the "Chi-square periodgram" method. (See Non-patent document)

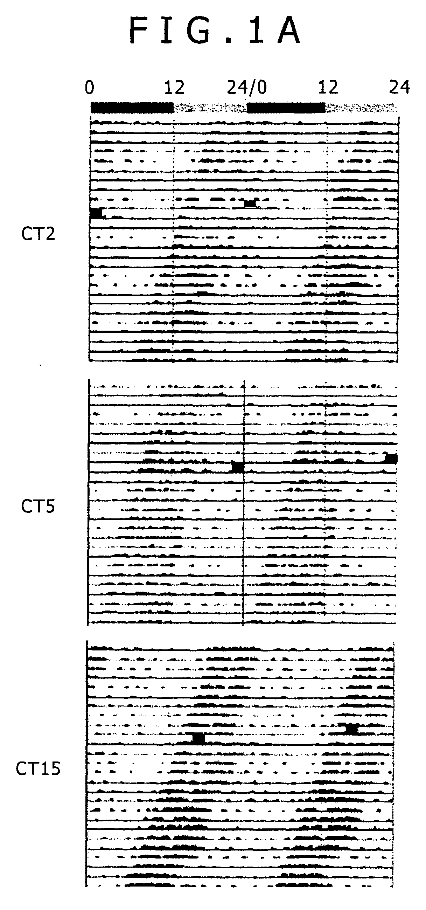

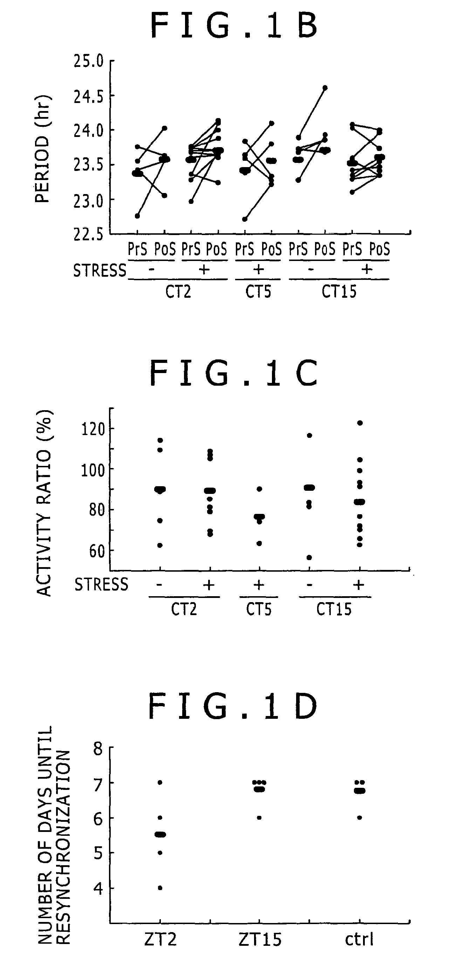

Fig. 1A is an actogram showing how a mouse changes its movement in one day under stress. Numerals at the top of Fig. 1 show the time of measurement. The black and gray lines below the top show the periods of bright and dark environments, respectively, which the mouse experienced before it began free run. The second and following rows in Fig. 1 show the actograms for two days, with each point representing the number of movements detected.
In Fig. 1, "CT2", "CT5", and "CT15" denotes respectively 2 o'clock, 5 o'clock, and 15 o'clock of circadian time. The circadian time denotes the mouse's subjective time. Since a mouse is nocturnal, the bright environment starts at 0 o'clock of circadian time if it experiences bright and dark environments alternately at an interval of 12 hours. Alternatively, the time at which a mouse begins to reduce its movement (or begins to sleep) due to the circadian rhythm is regarded as 0 o'clock if the bright and dark environments are switched at an interval of 24 hours.
Each actogram has two parts which are blotted out in black. They represent the time zone in which stress was applied. That is, the actograms of "CT2", "CT5", and "CT15" respectively show that stress was applied at 1-2 o'clock, 4-5 o'clock, and 14-15 o'clock (circadian time) of the 20th and 21 st days after the mouse had been left in the dark for 24 hours every day.
It is noted from Fig. 1A that the mouse under stress did not change in its circadian rhythm.
Fig. 1B is a diagram which graphically represents the average values of the mouse's circadian cycle observed in the actogram in Fig. 1A. "Period (hr)" on the vertical axis denotes the mouse's circadian cycle (or the length of the mouse's subjective one day). "PrS" and "PoS" on the horizontal axis denote respectively the circadian cycle before stress application and the circadian cycle after stress application. "Stress" denotes the presence or absence of stress application. "CT2", "CT5", and "CT15" are defined as above.
It is noted from Fig. 1 B that there is very little change in the circadian cycle before and after stress application.
Fig. 1C is a graph showing the change of activity ratio which was observed before and after stress application. "Activity ratio (%)" on the vertical axis denotes the value (%) obtained by dividing the activity value for 3 days after stress application by the activity value for 10 days before stress application. "Stress" denotes the presence or absence of stress application. "CT2", "CT5", and "CT15" are defined as above.

It is noted from Fig. 1C that there is very little difference in the activity ratio between the group (control) without stress application and the group with stress application.

Fig. 1D is a graph showing the number of days until resynchronization after stress application. This graph is based on the experiment which is intended to investigate how many days are necessary for the mouse to resynchronize its circadian rhythm again when it is left in the bright environment and the dark environment alternately at an interval of 12 hours (from 8 to 20 0'clock for the bright environment) and then it is given stress and finally it is left in the bright environment 6 hours earlier (from 2 to 14 o'clock for the bright environment).

"Number of days until resynchronization" on the vertical axis denotes the days necessary for resynchronization after the change of the time at which the mouse is left in the bright environment. "ZT2" denotes the group which was given stress at 1-2 o'clock (Zeitgeber time). Likewise, "ZT15" denotes the group which was given stress at 14-15 o'clock. "ctrl" denotes the group (control) which was not given stress. Here, "Zeitgeber time" denotes the time of the timing system in which the bright environment starts at 0 o'clock when the bright environment and the dark environment are repeated at an interval of 12 hours.

It is noted from Fig. 1D that there is very little difference between ZT15 and control in the number of days until resynchronization although ZT2 lacks uniformity.

The results in Example 1 indicate that stress application changes very little the circadian rhythm of individual mouse tested.

Example 2

This example is intended to investigate how the amount of expression of clock genes varies in a circadian cycle when stress is applied.

Each mouse was given stress for one hour at a prescribed time and then had its organs (liver, heart, and kidney) collected at intervals of four hours starting 12 hours after stress application. The collected organ was homogenized for extraction of total RNA by means of Promega total SV RNA Isolation Kit (from Promega). The extracted total RNA was used to determine the amount of expression of mPer1, mPer2, and Bmall genes by quantitative real time RT-PCR.

The thus obtained data showing the change with time of the amount of expression was graphed for fitting by the "Cosine fitting curve" method which is based on the formula below.

$$S(\cos, X) = \frac{1}{N} \sum_{i=1}^{N} \left( \sqrt{2} \cos(2\pi(ti - b)/24) \times \left( \frac{Xi - \overline{X}}{\phi_x} \right) \right)$$

$$\phi_x = \sqrt{\sum_{i=1}^{N} \frac{(Xi - \overline{X})^2}{N}}$$

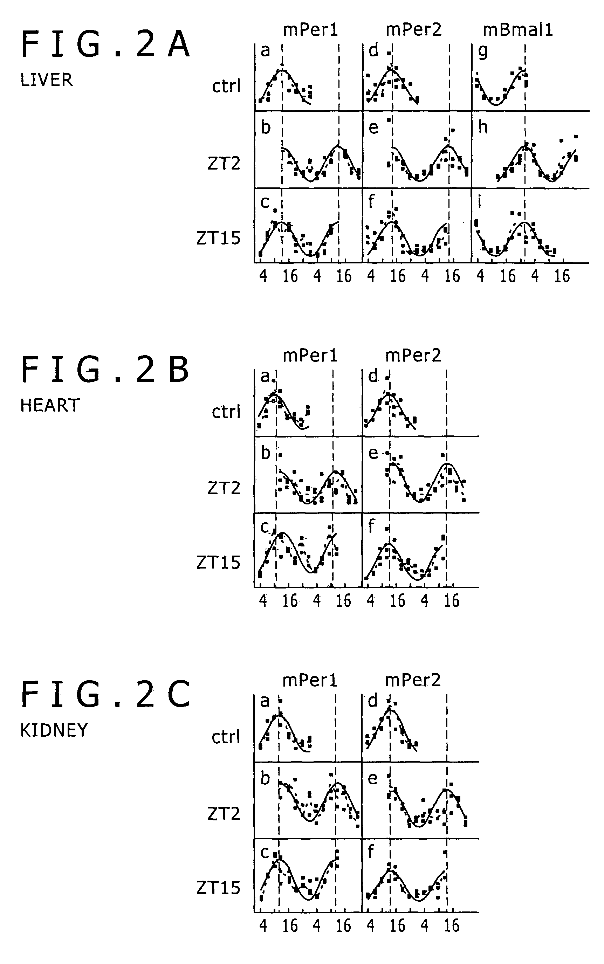

The results are shown in Figs. 2A to 2C.

Fig. 2A is a graph showing how the amount of expression of clock genes in the liver varies in a circadian cycle. Fig. 2B is a graph showing how the amount of expression of clock genes in the heart varies in a circadian cycle. Fig. 2C is a graph showing how the amount of expression of clock genes in the kidney varies in a circadian cycle.

In Figs. 2A to 2C, "mPer1", "mPer2", and "mBmal1" respectively denote how mPer1, mPer2, and mBmal1 change in the amount of expression in a circadian cycle. Incidentally, the prefix "m" attached to each gene name stands for mouse (Mus musculus). Moreover, in Figs. 2A to 2C, "ctrl" denotes the group which was not given stress (control), "ZT2" denotes the group which was given stress at 1-2 o'clock in Zeitgeber time, and "ZT15" denotes the group which was given stress at 14-15 o'clock in Zeitgeber time. The numerals on the horizontal axis represent the time at which the organs were collected.

It is noted from Figs. 2A to 2C that each clock gene varies in the amount of expression but there is very little difference in the phase shifting of the circadian rhythm between the group ("ZT2" and "ZT15"), which was given stress, and the group ("ctrl"), which was not given stress.

Thus, the results in Example 2 indicate that stress application causes very little change in the phase of the circadian rhythm in terms of the expression level of clock genes.

Example 3

This example is intended to investigate how the amount of expression of clock genes varies in each organ when stress is applied.

Each mouse was given stress for one hour at a prescribed time and then had its organs (liver, heart, lung, and kidney) collected at intervals of four hours starting 12 hours after stress application. The collected organ was homogenized for extraction of total RNA by means of Promega total SV RNA Isolation Kit (from Promega). The extracted total RNA was used to determine the amount of expression of each clock gene by quantitative real time RT-PCR.

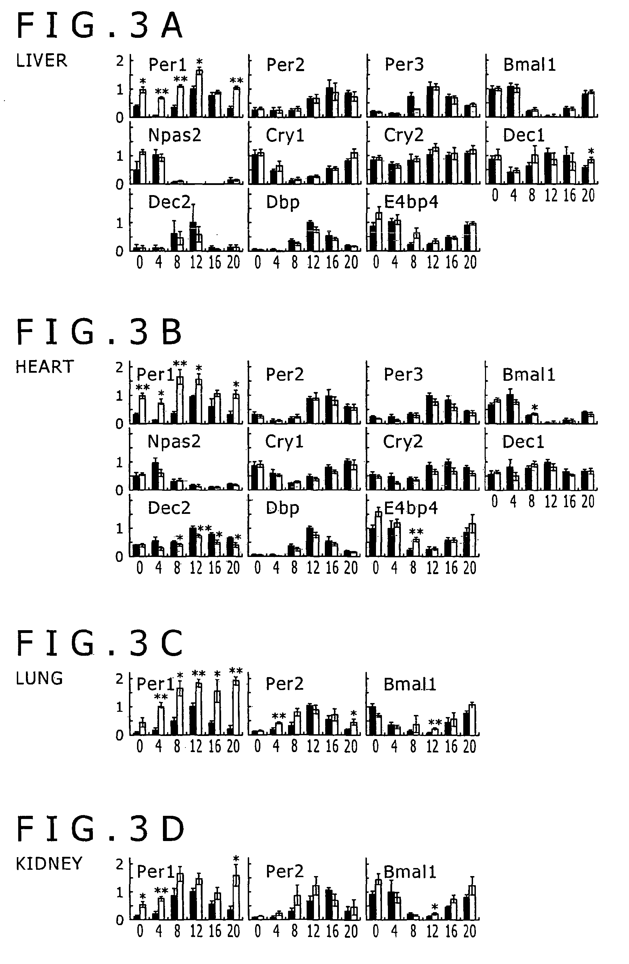

The results are shown in Figs. 3A to 3D.

Fig. 3A is a graph showing the amount of expression of clock genes in the liver. Fig. 3B is a graph showing the amount of expression of clock genes in the heart. Fig. 3C is a graph showing the amount of expression of clock genes in the lung. Fig. 3D is a graph showing the amount of expression of clock genes in the kidney.

In Figs. 3A to 2D, "Per1", "Per2", "Per3", "Bmal1", "Npas2", "Cry1", "Cry2", "Dec1", "Dec2", "Dbp", and "E4bp4" respectively denote the amount of expression of each clock gene. Moreover, black bars denote the amount of expression of clock genes in the control group which was not given stress, and white bars denote the amount of expression of clock genes in the group which was given stress. The numerals on the horizontal axis represent the time at which the organs were collected, and the numerals on the vertical axis denote the amount of expression (in relative values).

It is noted from Figs. 3A to 3D that stress application causes the amount of expression of Per1 gene to increase in each organ but does not cause the amount of expression of other clock genes to increase in each organ.

It is concluded from Examples 1 to 3 mentioned above that the circadian rhythm remains stable regardless of stress application as far as the activity rhythm of individuals or the expression level of clock genes is concerned, whereas the expression level of Per1 gene increases in peripheral tissues immediately after stress application.

The foregoing conclusion suggests the possibility of objective evaluation of stress by means of the expression level of Per1 gene in peripheral tissues which is used as an index.

Moreover, the results of experiments in the foregoing Examples suggest that the expression level of Per1 increases upon stress application by a new mechanism of transcriptional control which is different from the pathway through known promoters (such as E-box and CRE), e.g., Bmal/Clock-E-box pathway and CRE-CREB pathway.

Example 4

This example is intended to investigate the promoter of Per1 gene which is associated with stress response.

The first step was the in silico sequence analysis of the promoter region of Per1 gene on the basis of genome information. The results indicate that there are four sets of E-box and two sets of DBPE (Dbp-binding elements) in the sequence of the promoter region in all of human, rat, and mouse and that there are two sequences resembling GRE (glucocorticoid responsive elements) (which will be referred to as "GRE resembling sequence" hereinafter).

Incidentally, E-box exists not only in the promoter region of Per1 gene but also in the promoter regions of Per2 gene and Dbp gene. In addition, DBPE also exists in the promoter region of Per3 gene.

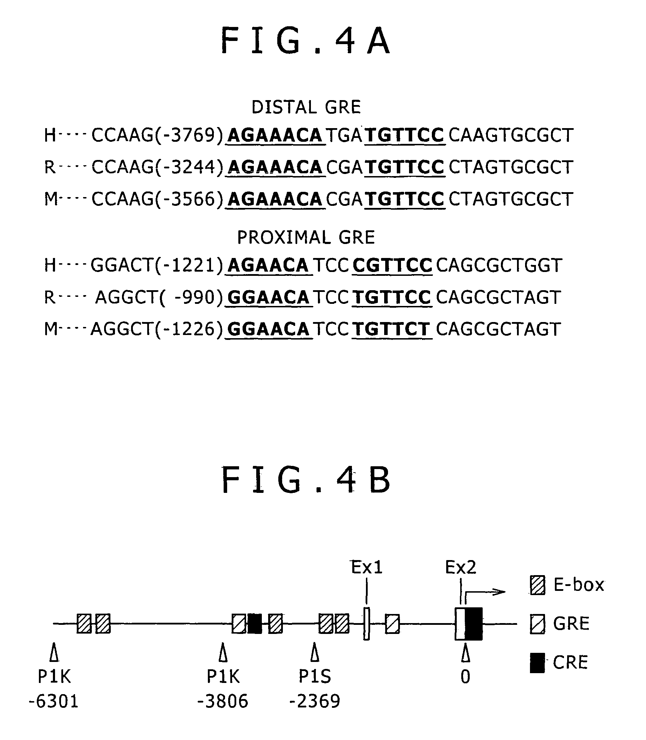

Fig. 4A is a diagram showing the GRE resembling sequence and the sequence in its vicinity. Fig. 4B is a schematic diagram showing the position of each promoter on the sequence.

In Figs. 4A and 4B, "Distal GRE" and "Proximal GRE" respectively denote the upstream sequence and downstream sequence of two sets of GRE resembling sequence. Also, "H", "R", and "M" denote the sequence of human, rat, and mouse, respectively. The underlined sequence is the one resembling GRE. "Ex1" and "Ex2" denote exon 1 and exon 2, respectively.

In Fig. 4B, "E-box", "GRE", and "CRE" respectively denote the position at which E-box exists, the GRE resembling sequence exists, and CRE exists in the promoter region of Per1 gene.

The second step was luciferase assay to investigate what part of the promoter region of Per1 is associated with stress response.

Luciferase is an enzyme protein that catalyzes bioluminescence. It can be used to estimate the level at which the expression of a specific gene is induced in the following manner. First, a recombinant gene is prepared in which the promoter region of a specific gene is joined to a gene that codes luciferase. Second, the recombinant gene is incorporated into cultured cells. Third, the gene is allowed to express so that the luciferase is allowed to express. Fourth, the intensity of bioluminescence due to luciferase is determined to obtain the level at which luciferase expresses.

An experiment was carried out based on the foregoing principle. It involves preparation of several kinds of DNA differing in the length of the promoter region of Per1 and determination of the expression level of Per1 in the presence of dexamethazone. The result thus obtained reveals what part of the promoter region of Per1 gene is associated with stress response. Incidentally, dexamethazone is synthetic glucocorticoid. It is known that the amount of glucocorticoid in blood increases when a living organism experiences stress.

The procedure of the experiment is outlined below.

(1) The first step is to prepare DNAs (P1F, P1K, and PIS), the first one having the entire length of the promoter region of Per1 gene and the second and third ones having the partial length of promoter region of Per1 gene, with the upstream side cut off. As shown in Fig. 4B, the position of truncation is 6301 bases upstream for P1F, 3806 bases upstream for P1K, and 2369 bases upstream for P1S, from the transcription starting region of Per1 gene.

(2) The second step is to prepare a recombinant vector by introduction of the foregoing DNA into luciferase luminescent vector, pGL3 basic (from Promega). The resulting recombinant vector has a promoter region of specific length and a region that codes the luciferase gene.

(3) The third step is to perform transfection of the recombinant vector (prepared in the second step (2)) into cultured cells NIH3T3 and addition of dexamethazone (0.1 $\mu$M). The cultured cells which have undergone transfection are isolated and subcultured for expression of luciferase. The subcultured cells are collected and dissolved 36 hours after transfection.

(4) The fourth step is to prepare a solution from the dissolved cells and measurement of the luminescence intensity by means of Dual Luciferase Assay System (from Promega). Thus it is possible to know the extent to which dexamethazone induces the expression of Per1 gene.

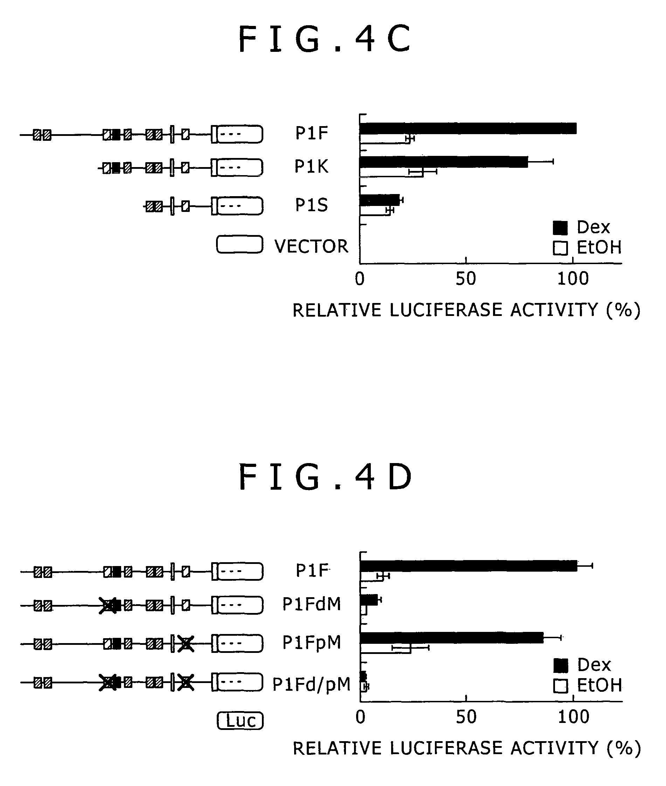

The results are shown in Fig. 4C.

Fig. 4C is a graph showing the results of luciferase assay. The abscissa (Relative Luciferase Activity) represents the intensity of luminescence in terms of relative value (%) compared with the intensity of luminescence assigned as 100 of the cultured cells incorporated with the entire length of the promoter region of Per1 gene.

"P1F" denotes the intensity of luminescence from cultured cells incorporated with the entire length of the promoter region of Per1 gene. "P1K" denotes the intensity of luminescence from cultured cells incorporated with the partial length of 3806 bases upstream from the transcription starting region of Per1 gene. "P1S" denotes the intensity of luminescence from cultured cells incorporated with the partial length of 2369 bases upstream from the transcription starting region of Per1 gene. "Vector" denotes the intensity of luminescence from cultured cells (control) incorporated with a blank vector. The right part of Fig. 4C is a schematic diagram illustrating the DNA which has been incorporated. "Dex" and "EtOH" respectively denote the intensity of luminescence from a sample containing dexamethazone and a sample (control) containing ethanol.

It is noted from Fig. 4C that dexamethazone contributes to a high intensity of luminance from the sample of "P1K" and a certain intensity of luminance from the sample of "P1S".

In view of the fact that the promoter sequence used in the sample of "P1K" has two GRE resembling sequences and the promoter sequence used in the sample of "P1S" has one GRE resembling sequence, the results of the experiment suggest that the GRE resembling sequence found in the in silico analysis could be associated with the stress response of Per1 gene.

Subsequent experiments were carried out to investigate whether expression is induced in the same way as shown in Fig. 4C when mutation is performed on both or either of the two GRE resembling sequences found by in silico analysis.

First, four DNA samples were prepared as follows:

DNA (P1F) with the promoter region of Per1 gene remaining intact over its entire length; DNA (P1FdM) with the GRE resembling sequence of P1F mutated in its upstream; DNA (P1FpM) with the GRE resembling sequence of P1F mutated in its downstream; and DNA (P1Fd/pM) with both of the GRE resembling sequences mutated. Each of the DNA samples was incorporated into the luciferase luminescent vector in the same way as mentioned above. The vector was transfected into cultured cells NIH3T3, which were subsequently given 0.1 $\mu$M of dexamethazone. Subcultured cells were collected and dissolved 36 hours after transfection. The resulting solution was tested for the intensity of luminance by means of Dual Luciferase Assay System (from Promega). This experiment reveals the extent to which dexamethazone induces the expression of Per1 gene.

The results are shown in Fig. 4D.

"P1F" denotes the intensity of luminescence from cultured cells incorporated with the entire length of the promoter region of Per1 gene. "P1FdM" denotes the intensity of luminescence from cultured cells incorporated with P1F having the GRE resembling sequence mutated in its upstream. "P1FpM" denotes the intensity of luminescence from cultured cells incorporated with P1F having the GRE resembling sequence mutated in its downstream. "P1Fd/pM" denotes the intensity of luminescence from cultured cells incorporated with P1F having the GRE resembling sequence mutated in both its upstream and its downstream.

It is noted from Fig. 4D that the sample of P1FpM containing dexamethazone gave a high intensity of luminescence, whereas the sample of P1FdM gave a little lower intensity of luminescence. This result suggests that the GRE resembling sequence existing in the promoter region of Per1, particularly the one existing in its downstream, is closely associated with stress response.

Example 5

This example is intended to investigate whether the GRE resembling sequence of Per1 gene joins to the glucocorticoid receptor (GR for short hereinafter) in NIH3T cells by using ChIP (Chromatin Immunoprecipitation) method.

First, NIH3T3 cells were treated with dexamethazone to induce the expression of mPer1 gene. The cells were given formaldehyde to fix the binding of protein and chromatin DNA. The chromatin DNA was fragmented by sonication and the resulting fragments underwent immunoprecipitation with anti-GR antibody. The resulting precipitates (as a composite of anti-GR antibody, GR, and chromatin DNA joining to GR) were recovered. The recovered precipitates were heated to remove the DNA-protein crosslinking. The desired sequence was amplified by PCR and the PCR product was identified by electrophoresis.

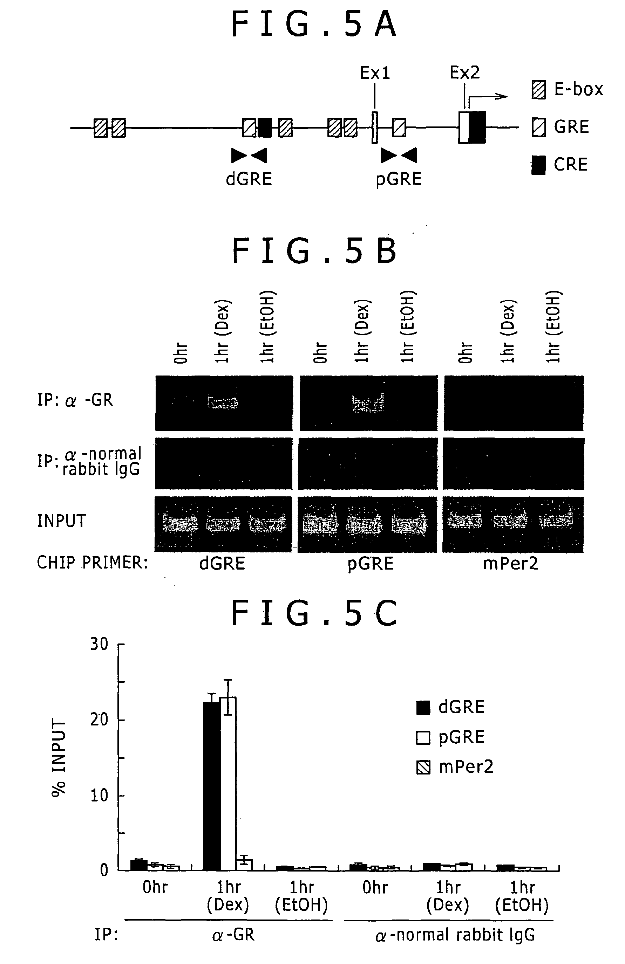

Fig. 5A is a schematic diagram illustrating the sequence of Per1 gene.

In Fig. 5A, triangular marks placed above "dGRE" and "pGRE" represent the sequence region corresponding to the primer used for PCR. In other words, the primer was so designed as to specifically amplify the region near the two GRE resembling sequences.

Fig. 5B is a photograph showing the electrophoresis for detection of the desired sequence by semi-quantitative RT-PCR which was performed on the immunoprecipitates obtained by ChIP method.

In Fig. 5B, "IP: α-GR" indicates the desired sequence detected by semi-quantitative RT-PCR after immunoprecipitation by means of anti-GR antibody. "IP: α-normal rabbit IgG" indicates the sequence (control) detected from immunoprecipitation by means of rabbit IgG. In addition, "input" indicates the desired sequence (control) detected by semi-quantitative RT-PCR without immunoprecipitation. "ChIP primer" in the lowermost row indicates the sequence of the primer used for semi-quantitative RT-PCR or the desired sequence which has been amplified. "dGRE" and "pGRE" respectively denote the amplified upstream sequence and downstream sequence out of the two GRE resembling sequences existing in the promoter region of Per1 gene. "mPer2" denotes the amplified mPer2 gene as control. "0hr" denotes the result which was obtained when formaldehyde was added immediately without treatment with dexamethazone. "1hr (Dex)" denotes the result obtained when formaldehyde was added one hour after treatment with dexamethazone. "1hr (EtOH)" denotes the result which was obtained when formaldehyde was added one hour after treatment with ethanol (for control).

Fig. 5C is a graph showing the result of the experiment to detect the desired sequence by quantitative real time RT-PCR that follows immunoprecipitation by ChIP method.

"%input" on the ordinate represents the amount of the desired DNA amplified in the PCR product. Other symbols are the same as those in Fig. 5B.

It is noted from Fig. 5B that the desired band was detected when the sequences indicated by "dGRE" and "pGRE" were amplified by PCR after treatment with dexamethazone and subsequent immunoprecipitation by means of anti-α GR antibody. (See the photograph of electrophoresis in the upper part of Fig. 5B.)

It is noted from Fig. 5C that PCR greatly amplifies the DNA containing the sequences indicated by "dGRE" and "pGRE" if it is carried out after treatment with dexamethazone and subsequent immunoprecipitation by means of anti-α GR antibody.

This result suggests that the glucocorticoid receptor (GR) joins to the two GRE resembling sequences out of the promoter region of Per1 gene.

Example 6

The same procedure as in Example 5 was repeated except that NIH3T cells were replaced by a liver collected from a mouse which had experienced stress.

A liver was collected from a mouse which had experienced stress for one hour and then rested for one hour. The collected liver was processed in the same way as in Example 5 to give a PCR product having the desired gene sequence.

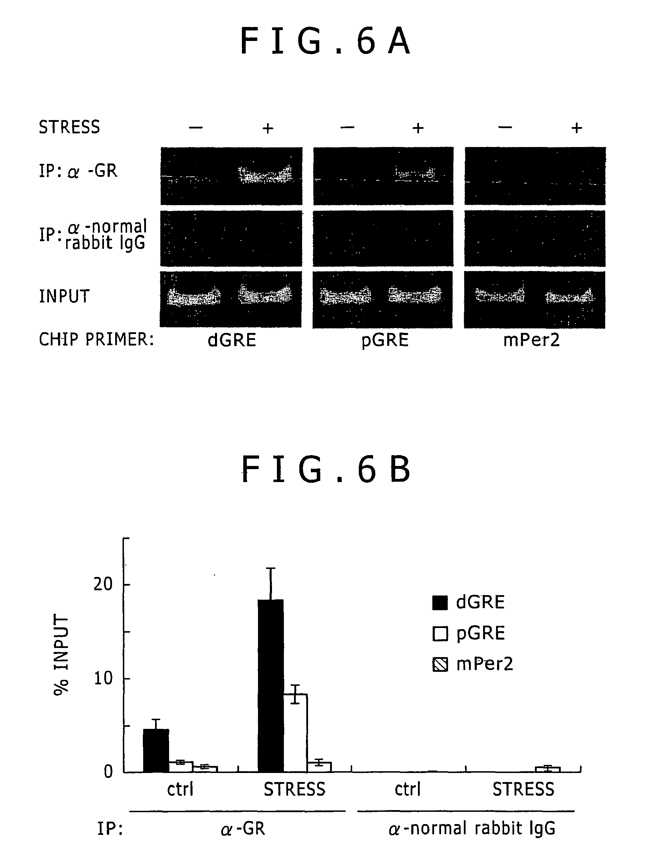

Fig. 6A is a photograph showing the electrophoresis for detection of the desired sequence by semi-quantitative RT-PCR which was performed on the immunoprecipitates obtained by ChIP method. Symbols in Fig. 6A are the same as those in Fig. 5B.

Fig. 6B is a graph showing the result of the experiment to detect the desired sequence by quantitative real time RT-PCR that follows immunoprecipitation by ChIP method. Symbols in Fig. 6B are the same as those in Fig. 5C.

It is noted form Figs. 6A and 6B that the glucocorticoid receptor (GR) joins to the two GRE resembling sequences out of the promoter region of Per1 gene even in the case where the mouse actually experiences stress.

It was found from the results shown in Figs. 6A and 6B that GR joined more to the upstream part of the two GRE resembling sequences existing in the promoter region of Per1 gene.

Example 7

This example is intended to investigate the amount of expression of Per1 in a mouse which was given corticosterone.

A mouse was given a prescribed amount of corticosterone, and one hour later, it had its liver extracted. The collected liver was homogenized for quantitative real time RT-PCR in the same way as in Example 2, and the amount of expression of clock genes was determined.

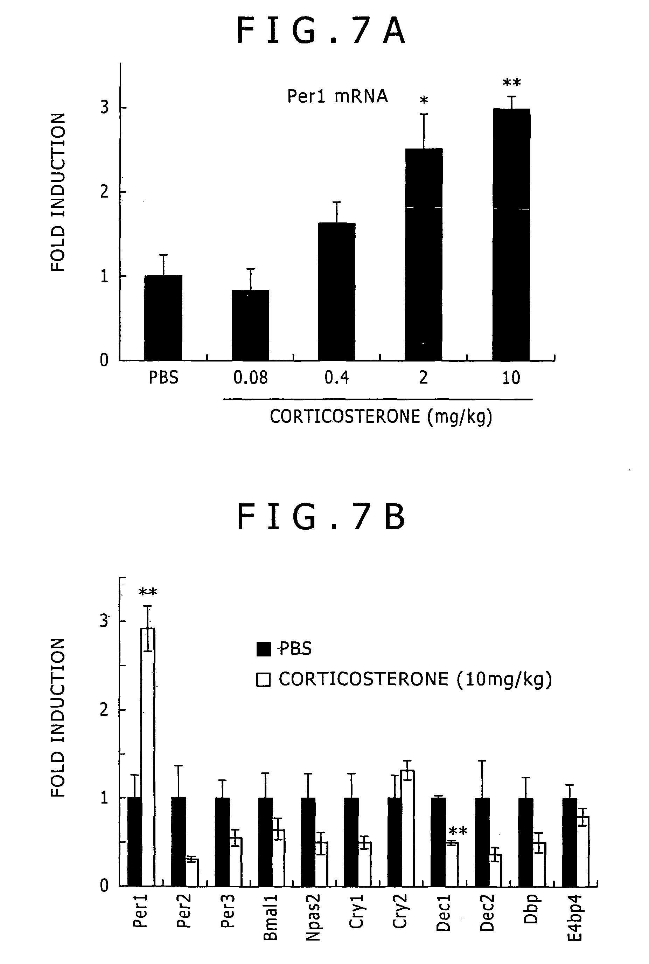

Fig. 7A is a graph showing the correlation between the dosage of corticosterone and the amount of expression of Per1 gene.

The abscissa represents the dosage (mg/kg) of corticosterone per 1 kg of the mouse's weight. "PBS" denotes the control which was given a phosphate buffer solution in place of corticosterone. "fold induction" on the ordinate represents the amount of expression of Per1 gene (in terms of a relative value, with the value for PBS being 1).

It is noted from Fig. 7A that the amount of expression of Per1 gene increases as the dosage of corticosterone increases.

Fig. 7B is a graph showing the amount of expression of each clock gene induced by administration of corticosterone (10 mg/kg).

The abscissa represents each clock gene. "fold induction" on the ordinate represents the amount of expression of each clock gene in terms of a relative value, with the value for PBS being 1.

It is noted from Fig. 7B that administration of corticosterone increases the amount of expression of only Per1 gene among clock genes. In other words, the result of this experiment suggests that the expression of Per1 gene due to stress application is induced by a mechanism which is entirely different from the adjustment of circadian rhythm.

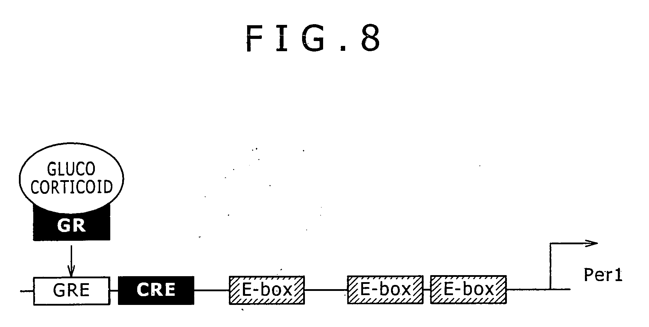

The results of experiments in Examples 1 to 7 mentioned above suggest the presence of the stress responsive mechanism as shown in Fig. 8.

It is noted from Fig. 8 that there exist not only E-box and CRE but also the GRE resembling sequence in the promoter region of Per1 gene.

Upon stress application, glucocorticoid is secreted from the adrenal cortex and carried to the entire body. Then, the glucocorticoid joins to GR existing in peripheral tissues. The resulting product joins to the GRE resembling sequence existing in the promoter region of Per1 gene. This is the mechanism to induce the expression of Per1 gene.

Incidentally, the sequences of E-box and CRE is not associated with the stress responsive mechanism. Therefore, they do not induce the expression of other clock genes having other sequences. Consequently, there is very little change in circadian rhythm (or internal body clock) even when stress is applied.

The method for stress evaluation according to the embodiment of the present invention can detect stress by using a new mechanism entirely different from the known stress responsive mechanism. The gene transcript and protein pertaining to the embodiment of the present invention can be used as the stress detecting marker. In addition, the polynucleotide and antibody pertaining to the embodiment of the present invention can be applied to DNA chips, protein chips, and stress evaluation kits.

The present invention contains subject matter related to Japanese Patent Application JP 2005-302589 filed in the Japanese Patent Office on October 18, 2005, the entire contents of which being incorporated herein by reference.

It should be understood by those skilled in the art that various modifications, combinations, sub-combinations and alternations may occur depending on design requirements and other factors insofar as they are within the scope of the appended claims or the equivalent thereof.

**Claims**

1. A method for stress evaluation which is designed to detect any increase in the expression level of Per1 gene.

2. The method for stress evaluation as defined in Claim 1 which is designed to detect any increase in the expression level in peripheral tissues.

3. Per1 gene which increases in the expression level when stress is applied.

4. The Per1 gene as defined in Claim 3 which increases in the expression level in peripheral tissues.

5. The Per1 gene as defined in Claim 3 which increases dependently on the glucocorticoid responsive element.

6. Polynucleotide which has the base sequence or part thereof of the Per1 gene defined in Claim 3.

**7.** Use of the transcript of the Per1 gene defined in Claim 3 as a marker for stress detection.

**8.** PER1 which is a protein coded by the Per1 gene defined in Claim 3.

**9.** An anti-PERI antibody which specifically joins to the PER1 defined in Claim 8.

**10.** Use of the PER1 defined in Claim 8 as a marker for stress detection.

# FIG.1A

# F I G . 1 B

# F I G . 1 C

# F I G . 1 D

FIG.2A
LIVER

FIG.2B
HEART

FIG.2C
KIDNEY

# FIG.3A

# FIG.3B

# FIG.3C

# FIG.3D

# F I G . 4 A

DISTAL GRE

H····CCAAG(-3769) **AGAAACA** TGA **TGTTCC** CAAGTGCGCT

R····CCAAG(-3244) **AGAAACA** CGA **TGTTCC** CTAGTGCGCT

M····CCAAG(-3566) **AGAAACA** CGA **TGTTCC** CTAGTGCGCT

PROXIMAL GRE

H····GGACT(-1221) **AGAACA** TCC **CGTTCC** CAGCGCTGGT

R···· AGGCT( -990) **GGAACA** TCC **TGTTCC** CAGCGCTAGT

M····AGGCT(-1226) **GGAACA** TCC **TGTTCT** CAGCGCTAGT

# F I G . 4 B

# FIG.4C

P1F

P1K

P1S

VECTOR

■ Dex
□ EtOH

0          50          100

RELATIVE LUCIFERASE ACTIVITY (%)

# FIG.4D

P1F

P1FdM

P1FpM

P1Fd/pM

Luc

■ Dex
□ EtOH

0          50          100

RELATIVE LUCIFERASE ACTIVITY (%)

# FIG. 5A

# FIG. 5B

# FIG. 5C

# F I G . 6 A

| STRESS | − | + | − | + | − | + |
|---|---|---|---|---|---|---|
| IP: α -GR | | | | | | |
| IP: α -normal rabbit IgG | | | | | | |
| INPUT | | | | | | |
| CHIP PRIMER: | dGRE | | pGRE | | mPer2 | |

# F I G . 6 B

■ dGRE
☐ pGRE
▨ mPer2

# FIG.7A

# FIG.7B

# FIG.8

**European Patent Office**

## EUROPEAN SEARCH REPORT

Application Number

EP 06 25 5175

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2005 143420 A (SONY CORP) 9 June 2005 (2005-06-09) * abstract * ----- | 1,2,7,10 | INV. C12Q1/68 |
| X | DATABASE EMBL [Online] EMBL SEQUENCE VERSION ARCHIVE 12 September 2005 (2005-09-12), "Homo sapiens hper1 gene for period1, complete cds" XP002421277 retrieved from EBI accession no. EMBL:AB030817 Database accession no. AB030817 * the whole document * ----- | 3-6,8 | |
| X | WO 99/12952 A (RES DEV FOUNDATION [US]) 18 March 1999 (1999-03-18) * claims 1-11 * * page 24, paragraph 2 - page 26; figure 2 * ----- | 3-6,8,9 | |
| X | US 2002/009730 A1 (CHENCHIK ALEX [US] ET AL) 24 January 2002 (2002-01-24) * the whole document * ----- | 1,2,7 | **TECHNICAL FIELDS SEARCHED (IPC)** C12Q |
| A | TARUSCIO D ET AL: "The human per1 gene: genomic organization and promoter analysis of the first human orthologue of the Drosophila period gene." GENE 8 AUG 2000, vol. 253, no. 2, 8 August 2000 (2000-08-08), pages 161-170, XP002421274 ISSN: 0378-1119 * the whole document * ----- | | |

-/--

The present search report has been drawn up for all claims

| Place of search | Date of completion of the search | Examiner |
|---|---|---|
| The Hague | 21 February 2007 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TAKAHASHI S ET AL: "Physical and inflammatory stressors elevate circadian clock gene mPer1 mRNA levels in the paraventricular nucleus of the mouse." ENDOCRINOLOGY NOV 2001, vol. 142, no. 11, November 2001 (2001-11), pages 4910-4917, XP002421323 ISSN: 0013-7227 * the whole document * | | |

TECHNICAL FIELDS SEARCHED (IPC)

The present search report has been drawn up for all claims

| Place of search | Date of completion of the search | Examiner |
|---|---|---|
| The Hague | 21 February 2007 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 06 25 5175

This annex lists the patent family members relating to the patent documents cited in  the above-mentioned European search report.
The members are as contained in the European Patent Office EDP file on
The European Patent Office is in no way liable for these particulars which are merely given for the purpose of information.

21-02-2007

| Patent document cited in search report | | Publication date | Patent family member(s) | | Publication date |
|---|---|---|---|---|---|
| JP 2005143420 | A | 09-06-2005 | NONE | | |
| WO 9912952 | A | 18-03-1999 | AU | 9380898 A | 29-03-1999 |
| US 2002009730 | A1 | 24-01-2002 | NONE | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000 A
- JP 275248 A

- JP 2005302589 A

**Non-patent literature cited in the description**

- **SOKOLOVE P.G. ; BUSHELL W.N.** *J Theor Biol.,* 1978, vol. 72, 131-160